# EUROPÄISCHE PATENTSCHRIFT

(11) **EP 3 689 392 B1**
(45) Veröffentlichungstag und Bekanntmachung des Hinweises auf die Patenterteilung: **20.03.2024**
(21) Anmeldenummer: 20157600.6
(22) Anmeldetag: 15.04.2010
(51) Int. Cl.: A61M 5/14, A61M 1/36, A61M 1/34

(54) **BEHANDLUNGSVORRICHTUNG ZUM ENTFERNEN VON BLUT AUS EINEM EXTRAKORPORALEN BLUTKREISLAUF**
TREATMENT APPARATUS FOR WITHDRAWING BLOOD FROM AN EXTRACORPOREAL BLOOD CIRCUIT
DISPOSITIF DE TRAITEMENT ÉLIMINANT LE SANG D'UN CIRCUIT SANGUIN EXTRACORPOREL

(30) Priorität: 23.04.2009 DE 102009018664; 10.06.2009 DE 102009024606; 10.06.2009 US 18565109 P
(43) Veröffentlichungstag der Anmeldung: 05.08.2020
(62) Teilanmeldung aus: 10714206.9
(73) Patentinhaber: Fresenius Medical Care Deutschland GmbH, 61352 Bad Homburg (DE)
(72) Erfinder: Günther, Götz, verstorben (DE); Manke, Joachim, 35792 Löhnberg (DE)
(74) Vertreter: Bobbert & Partner Patentanwälte PartmbB

(56) Entgegenhaltungen:
- DE-A1- 10 245 619
- US-A1- 2008 149 551

## Beschreibung

Die vorliegende Erfindung betrifft eine Blutbehandlungsvorrichtung gemäß dem Oberbegriff des Anspruchs 1.

Bei extrakorporaler Blutbehandlung ist großes Augenmerk zu legen auf das Erkennen und Beseitigen von eventuellen Gas- oder Lufteinschlüssen in einem zur Blutbehandlung verwendeten extrakorporalen Blutkreislauf.

Extrakorporale Blutkreisläufe sind üblicherweise Einweg-Artikel und werden nach ihrer Verwendung entsorgt. Dabei kann es aus verschiedenen Gründen von Vorteil sein, Flüssigkeiten aus dem extrakorporalen Blutkreislauf im Anschluss an eine Blutbehandlungssitzung zu entfernen. Zu diesen Gründen zählen Hygieneaspekte und die Kosten für das Entsorgen des gebrauchten extrakorporalen Blutkreislaufs.

Das Dokument DE10245619 beschreibt ein Vorrichtung zur Blutrückgabe aus einer Blutbehandlungsvorrichtung, vorzugsweise einer Dialysevorrichtung. Die in der Behandlungsvorrichtung enthaltene Substituatpumpe verdrängt mittels entsprechend geförderter Substituatflüssigkeit das Blut, bis in entsprechenden Detektoren festgestellt wird, dass statt Blut Substituatflüssigkeit in der Leitung nachströmt. Von diesem Zeitpunkt an wird das Blutvolumen kontrolliert weiterverdrängt, bis es den Leitungsauslass der entsprechenden Leitung erreicht hat. Eine Aufgabe der vorliegenden Erfindung ist, eine weitere Behandlungsvorrichtung zum Entfernen von Blut aus einem extrakorporalen Blutkreislauf nach Beenden einer Blutbehandlungssitzung anzugeben. Ferner soll eine Vorrichtung angegeben werden zum Erkennen und/oder Beseitigen von Luft, welche innerhalb eines extrakorporalen Blutkreislaufs vorliegt.

Die Aufgabe der vorliegenden Erfindung wird durch eine Vorrichtung mit den Merkmalen des Anspruchs 1 gelöst.

Eine "Blutbehandlungssitzung" wie hier diskutiert kann beispielsweise eine Behandlung mittels Hämodialyse, Hämofiltration, Hämodiafiltration und/oder eines Zellseparationsverfahrens umfassen. Sie kann auf die Reinigung von Blut gerichtet sein.

Zum Durchführen einer solchen Blutbehandlung wird eine geeignete Behandlungsvorrichtung verwendet.

Eine erfindungsgemäße "Behandlungsvorrichtung" weist im Gebrauch wenigstens einen extrakorporalen Blutkreislauf oder eine Aufnahmevorrichtung für einen solchen, wenigstens eine Blutpumpe sowie wenigstens eine zweite Fördereinrichtung auf.

Ein "extrakorporaler Blutkreislauf weist wenigstens einen arteriellen Leitungsabschnitt und wenigstens einen venösen Leitungsabschnitt auf. Jeder dieser Leitungsabschnitte umfasst ein Leitungsinneres.

Der extrakorporale Blutkreislauf kann geeignet sein, mit dem Gefäßsystem eines Patienten verbunden zu werden. Der extrakorporale Blutkreislauf kann jedoch auch ein solcher sein, welcher bei der Behandlung des Patienten extrakorporal Blut führt, mit dem Gefäßsystem des Patienten jedoch nicht verbunden ist.

Unter dem "Gefäßsystem des Patienten" ist hier der Blutkreislauf des Patienten als anatomische Struktur zu verstehen, welcher u.a. *post partum* angelegte Fisteln, Shunts und dergleichen, ferner arterielle und venöse Leitungsstrukturen des Körpers und dergleichen umfasst.

Unter dem Begriff "Blutpumpe" ist eine Fördereinrichtung zu verstehen, die zum Fördern von Blut im extrakorporalen Blutkreislauf geeignet und vorgesehen (im Weiteren synonym verwendet für "konfiguriert") ist.

Ein "Patient" im Sinne der vorliegenden Erfindung kann ein Mensch oder Tier sein.

Eine "zweite Fördereinrichtung" ist zum Einbringen wenigstens einer Substituatflüssigkeit in den extrakorporalen Blutkreislauf geeignet und vorgesehen.

Eine "Substituatflüssigkeit" kann dabei beispielsweise jede zur Verwendung bei einer Blutbehandlung übliche Substituatflüssigkeit sein. Vorzugsweise ist die Substituatflüssigkeit eine bereits während der Blutbehandlungssitzung verwendete und somit über eine Fluidverbindung bereits in den extrakorporalen Blutkreislauf eingebundene oder einbringbare Lösung. Sie kann vorzugsweise eine isotone Kochsalzlösung sein, wie z.B. eine 0,9%-ige NaCl-Lösung. Unter der Substituatflüssigkeit kann beispielsweise auch ein z.B. mittels Perfusor über eine Zugabestelle eingebrachtes Medikament verstanden werden.

Unter der Substituatflüssigkeit kann - ungeachtet ihres Namens - erfindungsgemäß auch ein anderes Fluid als eine Flüssigkeit verstanden werden, deren Verwendung sich erfindungsgemäß ebenfalls anbietet.

Die erfindungsgemäße Vorrichtung umfasst den Schritt des Einbringens oder Eintragens von Flüssigkeit in den extrakorporalen Blutkreislauf, vorzugsweise durch Betreiben der zweiten Fördereinrichtung.

Die in den extrakorporalen Blutkreislauf eingebrachte oder eingetragene Substituatflüssigkeit kann unter Betreiben der Blutpumpe und/oder der zweiten Fördereinrichtung durch den extrakorporalen Blutkreislauf gefördert werden.

Mittels der im extrakorporalen Blutkreislauf geförderten Substituatflüssigkeit kann das im extrakorporalen Blutkreislauf nach der Blutbehandlungssitzung vorhandene Blut des Patienten gleichzeitig sowohl aus dem arteriellen Leitungsabschnitt als auch aus dem venösen Leitungsabschnitt des extrakorporalen Blutkreislaufs verdrängt werden.

Der Begriff "gleichzeitig", wie er hierin verwendet wird, bedeutet, dass sowohl das Fördern von Blut aus dem arteriellen Leitungsabschnitt als auch das Fördern von Blut aus dem venösen Leitungsabschnitt zu ein und demselben Zeitpunkt x und/oder über ein und dieselbe Zeitdauer y erfolgen kann.

"Gleichzeitig" kann erfindungsgemäß als "simultan" oder "im selben Moment" verstanden werden.

Das "gleichzeitige Verdrängen" von Blut sowohl aus dem arteriellen Leitungsabschnitt als auch aus dem venösen Leitungsabschnitt des extrakorporalen Blutkreislaufs bedeutet, dass das Blut mittels der im extrakorporalen Blutkreislauf geförderten Substituatflüssigkeit zum gleichen Zeitpunkt x bzw. im gleichen Zeitraum y aus beiden Leitungsabschnitten des extrakorporalen Blutkreislaufs aus dem extrakorporalen Blutkreislauf entfernt wird.

Das im extrakorporalen Blutkreislauf nach einer Blutbehandlungssitzung vorhandene Blut wird aus dem extrakorporalen Blutkreislauf entfernt und kann verworfen werden.

Vorteilhafte Weiterbildungen der erfindungsgemäßen Vorrichtung sind jeweils Gegenstand der Unteransprüche.

Obwohl die vorliegende Beschreibung an verschiedenen Stellen auf eine Behandlungsvorrichtung für eine Dialysebehandlung, wie beispielsweise eine Hämodialyse, Bezug nimmt, ist die vorliegende Erfindung keinesfalls auf diese Vorrichtung beim Entfernen von Blut aus einem extrakorporalen Blutkreislauf nach einer Dialysebehandlung beschränkt.

Vielmehr ist die erfindungsgemäße Vorrichtung zum Entfernen oder Verdrängen von Blut aus jedem extrakorporalen Blutkreislauf, wie er zur Verwendung in einer Vielzahl von extrakorporalen Blutbehandlungen oder allen extrakorporalen Blutbehandlungen bekannt ist, geeignet.

Der extrakorporale Blutkreislauf kann vorzugsweise mittels eines so genannten DoubleNeedle-Zugangs oder mittels eines so genannten SingleNeedle-Zugangs mit dem Gefäßsystem des Patienten konnektiert sein.

Unter einem "DoubleNeedle-Zugang" zum Gefäßsystem eines Patienten ist eine Konnektionsform zu verstehen, bei der jeweils eine arterielle Konnektionsnadel und eine venöse Konnektionsnadel mit dem Gefäßsystem des Patienten und mit dem extrakorporalen Blutkreislauf bzw. dessen arteriellen und venösen Abschnitt konnektiert sind.

Obwohl die vorliegende Beschreibung beispielhaft anhand eines DoubleNeedle-Zugangs beschrieben ist, ist sie nicht auf eine solche Ausführung beschränkt.

Die erfindungsgemäße Vorrichtung kann ebenso unter Verwenden eines SingleNeedle-Zugangs benutzt werden. Dabei kann gleichzeitig aus dem arteriellen Leitungsabschnitt und dem venösen Leitungsabschnitt in Richtung zur einzigen Nadel zur Konnektion mit dem Patienten rückgeführtes Blut in einer "Y"-förmigen Aufgabelung aufeinander treffen.

Zu diesem Zweck können die "Y"- förmige Aufgabelung und/oder die Konnektionsnadel zum Gefäßsystem des Patienten geeignet vorgesehen bzw. ausgestaltet sein, beispielsweise durch geeignete Abmessungen des Querschnitts der Komponenten und/oder durch Anordnen von strömungsberuhigenden Elementen, Umströmungselementen oder dergleichen.

In einer bevorzugten Ausführungsform wird Blut unter gleichzeitigem Betreiben der Blutpumpe und der zweiten Fördereinrichtung aus dem extrakorporalen Blutkreislauf gefördert.

Bevorzugt kann das Blut sowohl gleichzeitig als auch mit gleichem relativem Fortschritt sowohl aus dem arteriellen Leitungsabschnitt als auch dem venösen Leitungsabschnitt des extrakorporalen Blutkreislaufs gefördert werden.

Das Blut kann jedoch alternativ gleichzeitig, jedoch mit unterschiedlichem relativem Fortschritt sowohl aus dem arteriellen Leitungsabschnitt als auch dem venösen Leitungsabschnitt des extrakorporalen Blutkreislaufs gefördert werden.

Der Begriff (relativer) "Fortschritt" kann sich hierbei auf die Bewegung bzw. das Fortschreiten des Bluts beziehen, z.B. eines Blutabschnitts, einer Blutfront und dergleichen in einem Leitungsabschnitt (im arteriellen oder venösen Leitungsabschnitt) des extrakorporalen Blutkreislaufs in Bezug auf Patienten bzw. einen Zugang zum Gefäßsystem desselben.

Der Begriff "relativ" bezeichnet dabei das Fortschreiten des Bluts im Verhältnis zu der zu durchströmenden Länge des jeweiligen Leitungsabschnitts des extrakorporalen Blutkreislaufs. D.h., dass das Fortschreiten des Bluts im arteriellen Leitungsabschnitt und im venösen Leitungsabschnitt relativ gesehen beispielsweise gleichschnell erfolgen kann. Da ein Leitungsabschnitt länger, mit größerem Durchmesser, mit Verzweigungen usw. ausgestaltet sein kann als der andere Leitungsabschnitt, entspricht das relative Fortschreiten demnach nicht dem absoluten Fortschreiten zum Beispiel bzgl. der Angabe exakter Volumen- oder Zentimeterangaben. Vielmehr kann das Fortschreiten beispielsweise als Prozentsatz eines von Blut bereits entleerten Leitungsabschnitts oder noch zu entleerenden Leitungsabschnitts angegeben werden.

Das gleichschnelle Fortschreiten des Bluts in unterschiedlich ausgestalteten Leitungsabschnitten kann beispielsweise über unterschiedliche Rotationsgeschwindigkeiten der Fördereinrichtungen erreicht werden. Das gleichschnelle Fortschreiten kann beispielsweise auch durch die Menge und die Lage der Zugabestelle der Substituatflüssigkeit und dergleichen erreicht werden.

Der arterielle Leitungsabschnitt und/oder der venöse Leitungsabschnitt des extrakorporalen Blutkreislaufs können während der Durchführung des Verfahrens, das nicht beansprucht wird, mit einem Patienten konnektiert sein. Eine solche Konnektion kann, wie vorstehend angegeben, in Form eines DoubleNeedle- oder eines SingleNeedle-Zugangs ausgeführt sein.

Das aus dem extrakorporalen Blutkreislauf verdrängte Blut kann vorzugsweise im Wesentlichen oder vollständig in das Gefäßsystem des Patienten rückgeführt werden. Im Falle z.B. eines SingleNeedle-Zugangs kann das Blut über einen gemeinsamen Zugang in das Gefäßsystem des Patienten erneut eingebracht bzw. rückgeführt werden.

In einem Verfahren, das nicht beansprucht wird, wird das Blut solange gefördert, bis eine Phasengrenze bzw. ein Mischbereich zwischen Blut und Substituatflüssigkeit eine Erfassungseinrichtung erreicht, welche in oder an einem Abschnitt des extrakorporalen Blutkreislaufs der Behandlungsvorrichtung angeordnet ist.

Die Erfassungseinrichtung kann vorzugsweise zum Erfassen einer qualitativen Änderung des Inhalts des (Leitungsinneren des) extrakorporalen Blutkreislaufs ausgestaltet und vorgesehen sein.

Eine "Erfassungseinrichtung" kann z.B. ein - vorzugsweise optischer - Sensor sein, der eine, z.B. optische, Änderung des Inhalts des Leitungsinneren oder eine Eigenschaft des Inhalts erfasst.

Das Verfahren, das nicht beansprucht wird, kann vorzugsweise dann beendet werden, wenn die Erfassungseinrichtung beispielsweise das Auftreten der Phasengrenze bzw. des Mischbereichs zwischen Blut und Substituatflüssigkeit erkennt.

Besonders bevorzugter Weise sind wenigstens zwei Erfassungseinrichtungen, die gleich oder unterschiedlich ausgestaltet sein können, im extrakorporalen Blutkreislauf angeordnet. Insbesondere kann es von Vorteil sein, eine erste Erfassungseinrichtung im arteriellen Leitungsabschnitt und eine zweite Erfassungseinrichtung im venösen Leitungsabschnitt anzuordnen. Die beiden Erfassungseinrichtungen können beispielsweise in bzw. mit einem solchen Abstand zum jeweiligen Patientenzugang, zum Beispiel zur arteriellen Konnektionsnadel bzw. zur venösen Konnektionsnadel, angeordnet und vorgesehen sein, derart, dass, wenn die Erfassungseinrichtung jeweils das Auftreten einer Phasengrenze bzw. eines Mischbereichs zwischen Blut und Substituatflüssigkeit erkennt, das Blut im Wesentlichen bereits vollständig aus dem extrakorporalen Blutkreislauf in das Gefäßsystem des Patienten rückgeführt wurde.

In einem Verfahren, das nicht beansprucht wird, wird die Förderleistung oder die Förderrate der Blutpumpe unter Berücksichtigung wenigstens eines Parameters den extrakorporalen Blutkreislauf und/oder eine Förderleistung oder eine Förderrate der zweiten Fördereinrichtung betreffend, gesteuert oder geregelt.

Die Förderleistung oder die Förderrate der Blutpumpe können beispielsweise durch Steuern oder Regeln einer Rotationsgeschwindigkeit der Blutpumpe eingestellt werden.

Die Förderleistung oder die Förderrate der zweiten Fördereinrichtung kann einem vorgegebenen Wert bzw. vorgegebenen Werten entsprechen und beispielsweise in einer geeigneten Einrichtung im oder am extrakorporalen Blutkreislauf hinterlegt und auslesbar sein und/oder kontinuierlich erfasst und - vorzugsweise in Echtzeit - zum dynamischen Steuern bzw. Regeln der Förderleistung oder Förderrate der Blutpumpe eingesetzt werden.

Alternativ oder ergänzend ist in einer weiteren erfindungsgemäßen Ausführungsform vorgesehen, eine Förderleistung oder eine Förderrate der zweiten Fördereinrichtung unter Berücksichtigung wenigstens eines Parameters den extrakorporalen Blutkreislauf und/oder eine Förderleistung oder eine Förderrate der Blutpumpe betreffend, zu steuern oder zu regeln. Eine Steuerung oder Regelung der zweiten Fördereinrichtung bzw. einer Förderleistung oder einer Förderrate derselben kann analog der vorstehend angegebenen Vorgehensweise ausgeführt werden.

Unter dem Begriff "Parameter" wie vorstehend verwendet kann eine Größe und/oder Eigenschaft zu verstehen sein, welche einer Komponente des extrakorporalen Blutkreislaufs und/oder der zweiten Fördereinrichtung zugeordnet werden kann, beispielsweise ein Volumen, z.B. das Volumen oder die Länge des Leitungsinneren eines Leitungsabschnitts des extrakorporalen Blutkreislaufs, eine Förderrate der zweiten Fördereinrichtung, eine Geschwindigkeit, eine Förderrichtung und dergleichen.

In einer weiter bevorzugten Ausführungsform wird durch Betreiben der zweiten Fördereinrichtung eine vorbestimmte Menge an Substituatflüssigkeit in das Leitungsinnere des extrakorporalen Blutkreislaufs eingebracht.

Eine "vorbestimmte Menge an Substituatflüssigkeit bzw. eine vorbestimmte Substituatflüssigkeitsmenge" kann einem bestimmten Fördervolumen und/oder einer bestimmten Förderstrecke des zu fördernden bzw. zu verdrängenden Bluts entlang des Leitungsinneren des extrakorporalen Blutkreislaufs entsprechen. Das Einbringen einer vorbestimmten Menge an Substituatflüssigkeit kann beispielsweise durch Betreiben einer hochgenau dosierenden Membranpumpe erfolgen.

Die Menge an Substituatflüssigkeit kann vorzugsweise beispielsweise in einer Steuereinrichtung hinterlegt und/oder eingebbar sein.

In einem Verfahren, das nicht beansprucht wird, erfolgt mittels eines Lufterkennungsdetektors bzw. ABD (Air Bubble Detector), wie er aus dem Stand der Technik bekannt ist und auch weiter unten noch erläutert wird, und welcher in einen arteriellen Schenkel bzw. Leitungsabschnitt eines extrakorporalen Blutkreislaufs oder eines Schlauchsets eingefügt ist, eine Lufterkennung. Es wird darauf hingewiesen, dass der Begriff "Luft" hier nicht einschränkend verstanden werden soll. Die vorliegende Erfindung betrifft gleichermaßen jede Art von Gas.

Dabei kann eine Warnung beim Erkennen von Luft im arteriellen Leitungsabschnitt ergehen. Unabhängig hiervon kann die Blutförderung angehalten und/oder weitere Maßnahmen ergriffen werden.

In dieser Situation ist es insbesondere möglich, den Fehler, der zur Luftinfusion geführt hat, zu beheben, sei es automatisch oder durch Personal bzw. den Anwender. Die Warnung kann überbrückt werden, um die Förderung erneut aufzunehmen.

Auf diese Weise kann die Menge der eingetretenen Luftmenge und des möglicherweise erzeugten Blutschaumvolumens durch rasches Eingreifen vorteilhaft minimiert werden.

Alternativ oder ergänzend kann bei Erkennen von Lufteinschluss oder bei Ausgabe einer Warnung aufgrund von erkanntem Lufteinschluss eine Luftentfernung im arteriellen Leitungsabschnitt erfolgen, im Folgenden auch als arterielle Luftentfernung bezeichnet. Das dabei eingesetzte Verfahren, das nicht beansprucht wird, kann einer venösen Luftentfernung gleich sein.

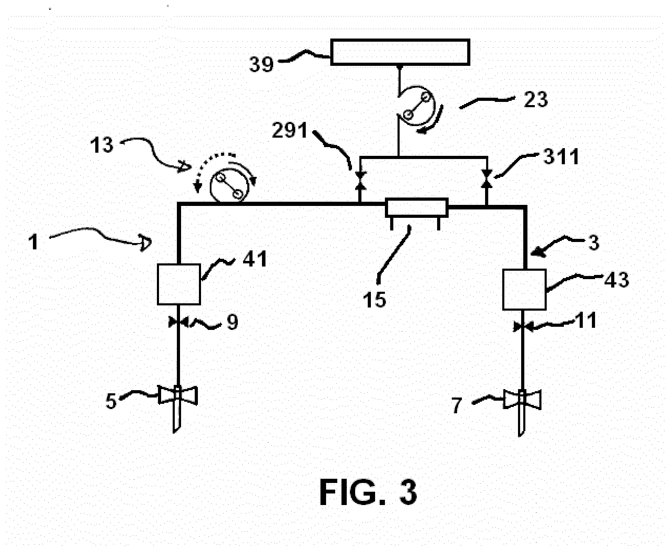

Die arterielle Luftentfernung kann sich in ihrer Anwendung für Onlinebehandlung und Standardbehandlung unterscheiden:
Bei der Onlinebehandlung wird der Patient arteriell dekonnektiert und statt des Patienten ein externes Behältnis, z.B. ein NaCl-Beutel, am arteriellen Patientenschlauch angeschlossen. Anschließend wird - vorzugsweise nach entsprechenden Sicherheitsabfragen - mit Hilfe der Substituatpumpe Substituat aus der Hydraulik - oder einer anderen Quelle - vorzugsweise über das Prädilutions-Ventil in den arteriellen Leitungsabschnitt des Schlauchsystems gefördert. Dies geschieht derart, dass die dort befindliche Luft in das angeschlossene Behältnis verdrängt wird. Dies ist auch in Fig. 3 dargestellt. Ist das Schlauchsystem luftfrei, wird das Behältnis dekonnektiert und der Patient erneut mit dem arteriellen Patientenschlauch oder Leitungsabschnitt verbunden. Auf diese Weise gelangt und/oder verbleibt keine Luft in das extrakorporale Blutsystem.

U.a. bei der HD-Behandlung (Hämodialyse) kann eine Luftentfernung allerdings auch wie folgt vonstatten gehen: Bei erkanntem Lufteinschluss wird der Patient sowohl arteriell als auch venös dekonnektiert. Beide Patientenschläuche oder Leitungsabschnitte werden an ein Behältnis - vorzugsweise den zuvor erwähnten NaCl-Beutel - oder verschiedene Behältnisse angeschlossen. Anschließend wird - vorzugsweise nach vorherigen Sicherheitsabfragen - durch Rückwärtsförderung durch die Blutpumpe Inhalt des Behältnisses, z.B. NaCl, in das venöse Blutschlauchsystem bzw. den venösen Leitungsabschnitt gesaugt und damit die im arteriellen Patientenschlauch befindliche Luft in das Behältnis gefördert. Erkennt der Anwender oder eine entsprechende Einrichtung, dass das Schlauchsystem luftfrei ist (hierzu bietet sich eine zusätzliche Kontrolle des venösen Zweigs bzw. Schenkels an), so wird das Behältnis dekonnektiert und wiederum der Patient mit den Patientenschläuchen angeschlossen. Auch hierbei gelangt und/oder verbleibt die Luft nicht in das extrakorporale Blutsystem.

Neben den beiden letzten der vorstehend beschriebenen Verfahren, die nicht beansprucht sind und welche sich auch als "Vorwarnfunktionen für arteriell angesaugte Luft" bezeichnen lassen, kann unter Einsatz wenigstens eines Lufterkennungsdetektors (im Folgenden auch kurz: ABD) auch ein Verfahren, das auch nicht beansprucht ist, zur sicheren Rückwärtsförderung im extrakorporalen System durchgeführt werden. Bei diesem Verfahren ist eine sichere Rückwärtsförderung von Blut mittels der Blutpumpe vorteilhaft auch bei konnektiertem Patienten möglich.

Erkennen der ABD und die entsprechende Steuerung dabei arteriell Luft, so wird ein arterieller Luftalarm ausgelöst. Dieser kann zum unverzüglichen Schließen der arteriellen Patientenschlauchklemme und/oder zum Anhalten der Blutförderung führen oder darin bestehen. Dabei kann eine wie oben beschriebene Luftentfernung erfolgen. Alternativ kann die Flussrichtung umgekehrt werden. Die Luft kann auf diese Weise vorteilhaft über die venöse Tropfkammer abgeschieden werden.

Vorzugsweise wird hierbei mittels ABD und der entsprechenden Steuerung überprüft, dass sich auch im jeweiligen Schlauchabschnitt keine Luft befindet. Auf diese Weise wird vorteilhaft vermieden, dem Patienten Luft zu infundieren.

Dabei kann folgendes nicht beanspruchtes Verfahren eingesetzt werden: Wird vom Anwender (oder automatisch) eine Flussumkehr angefordert, so wird zunächst bei aktivierter Luftwarnung noch ein definiertes Volumen (vorzugsweise mindestens das Volumen des Blutsystems bis hinter den jeweiligen ABD) weiter in die bisherige Förderrichtung gefördert, bevor die eigentliche Umkehr der Flussrichtung erfolgt bzw. eingeleitet wird. Vorzugsweise soll diese nur zulässig sein, wenn das gesamte Vorvolumen ohne Luftwarnung gefördert worden ist. Vorzugsweise hat der Anwender keine Möglichkeit der Manipulation oder des Eingreifens zwischen Luftwarnung und Umkehr der Flussrichtung. Mittels des vorstehend erläuterten Verfahrens, das nicht beansprucht wird, lässt sich vorteilhaft auch ein Fistelfluss bestimmen.

Mittels des im arteriellen Schenkel eingesetzten ABD ist ferner eine sog. simultane Reinfusion möglich, wie im Folgenden beschrieben. Dabei soll bevorzugt sein, die Einleitung von Substituat ohne zusätzliche Handlungen des Anwenders wie etwa Umstecken des Konnektors und dergleichen zwischen Prä- und Postdilution umschalten zu können.

Bei der simultanen Reinfusion verbleiben beide Konnektionsnadeln im Gefäßsystem des Patienten. Im venösen Teil des Schlauchsystems wird das Blut durch Fördern von Substituat über den Prädilutionsport verdrängt, bis der venöse optische Detektor kein Blut mehr erkennt.

Vorzugsweise soll die Blutpumpe gleichzeitig hierzu rückwärts laufen. Die Blutpumpe kann dabei vorzugsweise mit sehr niedriger Rate oder Geschwindigkeit laufen.

Ein Teil des Substituats wird hierdurch in den arteriellen Teil des Systems gefördert und verdrängt auch dort das Blut zum Patienten hin. Beide ABDs sind dabei aktiv. Die arterielle Förderung kann - da es sich um vergleichsweise kleine oder sehr kleine Volumina handelt - volumengesteuert angehalten werden. Vorzugsweise kann ein im ABD enthaltener optischer Sensor (OD) hierzu verwendet werden.

Bei diesem Verfahren, das nicht beansprucht wird, kann bei Auftreten eines arteriellen Luftalarms vorzugsweise nur die Blutpumpe angehalten werden. Die venöse Reinfusion kann dabei weiter erfolgen. Ein hierbei ggf. in Kauf zu nehmender Blutverlust beträgt weniger als 30 ml und ist damit vernachlässigbar bzw. führt nicht zu einer nennenswerten Beeinträchtigung des Patienten oder seines Befindens.

In bestimmten Verfahren, die nicht beansprucht sind, umfasst die Blutrückgabe bzw. Reinfusion von im extrakorporalen Blutkreislauf nach Beendigen der Blutbehandlung vorhandenen Blut über die arterielle Patientenleitung bzw. den arteriellen Leitungsabschnitt oder Schenkel den Einsatz eines Gerinnselfängers. Der Gerinnselfänger ist oder wird erfindungsgemäß derart angeordnet, um von Blut durchströmt zu werden, welches mittels der arteriellen Patientenleitung dem Patienten zugeführt oder auf andere Weise aus dem extrakorporalen Blutkreislauf entfernt wird.

Der Gerinnselfänger kann in manchen erfindungsgemäßen Ausführungsformen manuell in einen entsprechenden blutführenden arteriellen Abschnitt des extrakorporalen Blutkreislaufs eingefügt werden. Dies kann beispielsweise zwischen einem Abschnitt des arteriellen Leitungsabschnitts und der arteriellen Konnektionsnadel erfolgen. Der Gerinnselfänger kann beispielsweise zwischen einem, insbesondere ursprünglich vorhandenen, Schlauchende des arteriellen Leitungsabschnitts und einem, insbesondere ursprünglich vorhandenen, Ende des flexiblen Schlauchabschnitts der arteriellen Konnektionsnadel eingefügt werden, beispielsweise nach einfachem, üblichem Dekonnektieren des arteriellen Leitungsabschnitts vom flexiblen Schlauchabschnitt der arteriellen Konnektionsnadel. In bestimmten erfindungsgemäßen Ausführungsformen erfolgt das Einsetzen des Gerinnselfängers nach Abschluss der Blutbehandlung.

Der Einsatz des Gerinnselfängers umfasst in manchen erfindungsgemäßen Ausführungsformen einen oder mehrere der folgenden Schritte:
a) Der Gerinnselfänger, der ggf. zunächst aus einer sterilen Umverpackung entnommen werden muss, wird mit einer geeigneten Flüssigkeit, vorzugsweise einer Kochsalzlösung, gefüllt, sofern dies noch nicht der Fall ist.
b) Die Reinfusion und/oder die Blutförderung durch die Blutpumpe wird durch entsprechende Ansteuerung der Behandlungsvorrichtung unterbrochen. Dies erfolgt in manchen erfindungsgemäßen Ausführungsformen mittels Betätigen eines Soft-Keys der Behandlungsvorrichtung.
c) Am arteriellen Leitungsabschnitt und an der Schlauchleitung der arteriellen Konnektionsnadel (bzw. in deren Bereich) wird der Blutfluss unterbunden, vorzugsweise mittels zweier Patientenschlauchklemmen. Die Schlauchleitung der arteriellen Konnektionsnadel und der arterielle Leitungsabschnitt können daraufhin voneinander getrennt werden.
d) Der Gerinnselfänger wird mit der Schlauchleitung der arteriellen Konnektionsnadel verbunden.
e) Der Gerinnselfänger wird mit dem arteriellen Leitungsabschnitt verbunden.
f) Sofern vorhanden, werden die beiden Patientenschlauchklemmen (eine an der Schlauchleitung der arteriellen Konnektionsnadel und eine am arteriellen Leitungsabschnitt) geöffnet. In jedem Fall wird der unterbrochene Blutfluss wieder hergestellt.
g) Die Reinfusion und/oder die Blutförderung durch die Blutpumpe, welche vorübergehend unterbrochen wurde, wird erneut gestartet. Dies kann z.B. mittels eines Soft-Keys an der Behandlungsvorrichtung erfolgen.
h) Mittels der Behandlungsvorrichtung wird in manchen erfindungsgemäßen Ausführungsformen zunächst ein, vorzugsweise vorbestimmtes, Volumen über den arteriellen Leitungsabschnitt angesaugt. In bestimmten erfindungsgemäßen Ausführungsformen wird erst am Anschluss hieran die simultane Reinfusion mit gleichzeitiger Rückgabe über beide Leitungsabschnitte bzw. Patientenleitungen begonnen.

Die Reihenfolge der zuvor genannten Schritte a) bis h) kann dabei beliebig variiert werden im Rahmen dessen, was für den Fachmann als möglich erkennbar ist.

Der erfindungsgemäße Gerinnselfänger weist in bestimmten Ausführungsformen der hierin beschriebenen Erfindung eine Maschenweite von vorzugsweise 10 bis 200 Mikrometer (µm) sowie beliebige Zwischenwerte dieses Bereichs auf. In manchen erfindungsgemäßen Ausführungsformen beträgt die Maschenweite 40 oder 160 Mikrometer (µm).

Die erfindungsgemäße Aufgabe wird durch eine Behandlungsvorrichtung gemäß Anspruch 1 gelöst. Die mit dem nicht beanspruchten Verfahren erzielbaren Vorteile sind ungeschmälert auch mit der erfindungsgemäßen Behandlungsvorrichtung erzielbar.

Die erfindungsgemäße Behandlungsvorrichtung weist wenigstens einen extrakorporalen Blutkreislauf, welcher einen arteriellen Leitungsabschnitt und einen venösen Leitungsabschnitt aufweist, wenigstens eine Fördereinrichtung zum Einbringen und/oder Fördern eines Fluids im extrakorporalen Blutkreislauf sowie wenigstens eine Steuer- oder Regeleinrichtung auf, die geeignet und vorgesehen ist zum Ausführen oder Betreiben bzw. Steuern oder Regeln des nicht beanspruchten Verfahrens.

Die "Fördereinrichtung" zum Einbringen und/oder Fördern eines Fluids in den bzw. innerhalb des extrakorporalen Blutkreislaufs schließt eine an oder in dem extrakorporalen Blutkreislauf angeordnete Blutpumpe zum Fördern von Blut innerhalb des Leitungsinneren des extrakorporalen Blutkreislaufs ein. Die "Blutpumpe" kann z.B. als Schlauchpumpe oder Rollenpumpe oder jede andere geeignete Pumpe ausgeführt sein.

Ferner weist die erfindungsgemäße Behandlungsvorrichtung wenigstens eine zweite Fördereinrichtung zum Einbringen wenigstens einer Substituatflüssigkeit in das Leitungsinnere des extrakorporalen Blutkreislaufs zu deren Fördern im Leitungsinneren auf. Die zweite Fördereinrichtung kann eine peristaltische Pumpe, z.B. eine Schlauchpumpe oder eine Rollenpumpe, sein. Es kann jedoch auch ein anderer Pumpentyp, z.B. eine Membranpumpe, insbesondere eine hochgenau dosierende Membranpumpe eingesetzt werden.

Die zweite Fördereinrichtung kann Zuleitungen, beispielsweise eine Substituatflüssigkeitszuleitung, zu verschiedenen Aufbewahrungseinrichtungen, wie Substituatflüssigkeitsvorratseinrichtungen oder Vorratsbehältern mit Substituatflüssigkeit, aufweisen.

Die zweite Fördereinrichtung kann ferner wenigstens eine Steuer- oder Regeleinrichtung aufweisen, die ein automatisiertes Einbringen und/oder Fördern der wenigstens einen Substituatflüssigkeit, beispielsweise einer vorbestimmten Substituatflüssigkeitsmenge, erlaubt und (auch) hierzu vorgesehen ist.

Die zweite Fördereinrichtung und ihre Komponenten, insbesondere ihre Zuleitungen in den extrakorporalen Blutkreislauf, stellen vorzugsweise einen Teil des Schlauchsatzes bzw. Schlauchsystems für die extrakorporale Blutbehandlung dar. Sie können an oder im extrakorporalen Blutkreislauf vorgesehen sein. Sie können insbesondere mit diesem in geeigneter Weise, beispielsweise mit Hilfe von Klemmen, Anschlussstutzen oder dergleichen, verbunden sein.

Die zweite Fördereinrichtung kann sich alternativ auch innerhalb des extrakorporalen Blutkreislaufs befinden. Die zweite Fördereinrichtung kann Substituatflüssigkeit, z.B. über die Substituatflüssigkeitszuleitung, ansaugen.

Eine Substituatleitung bzw. Substituatflüssigkeitszuleitung ist vorzugsweise eine Zuleitung, die von einer Substituatquelle bzw. einer Substituatflüssigkeitvorratseinrichtung angeschlossen werden kann. Die Substituatleitung kann Bestandteil oder Zubehörteil eines Schlauchsatzes für die extrakorporale Blutbehandlung sein. Sie kann jedoch auch derart ausgestaltet sein, dass sie nicht unmittelbar Bestandteil des extrakorporalen Blutkreislaufs in dem Sinne ist, dass durch die Substituatleitung kein Blut strömt.

Die erfindungsgemäße Behandlungsvorrichtung kann ferner beispielsweise eine Behandlungseinrichtung zum Behandeln des Bluts des Patienten, wie einen oder mehrere Blutfilter und/oder einen oder mehrere Dialysatoren, beispielsweise einen Hämodialysator, und/oder einen oder mehrere Ab- oder Adsorber, aufweisen.

Die Behandlungsvorrichtung kann des Weiteren auch Behälter zum Aufbewahren von Fluiden, Elemente zum Einbringen der Fluide, wie beispielsweise Schlauchelemente und/oder Ventile, sowie weitere Einrichtungen, wie z.B. eine Tropfkammer zum Entfernen von Luft aus dem Blut während der Blutbehandlung und/oder Sensoren und/oder Detektoren zum Erfassen verschiedener relevanter Parameter, wie beispielsweise einen Druck im extrakorporalen Blutkreislauf, aufweisen.

Die einzelnen Komponenten können in Differentialbauweise miteinander verbunden werden, d.h. die Komponenten können additiv durch Fügetechnologie, z.B. durch Kleben und/oder Schweißen wie Kunststoffschweißen oder Laserschweißen, zusammengefügt werden. Die Komponenten können Bestandteil einer externen Funktionseinrichtung, wie beispielsweise einer Blutkassette, sein. Eine solche Blutkassette ist zum Beispiel in den von der Anmelderin der vorliegenden Erfindung am 23. April 2009 bzw. am 10. Juni 2009 beim DPMA hinterlegten Anmeldungen 10 2009 018 664.6 bzw. 10 2009 024 468.9 mit jeweils dem Titel "*Externe Funktionseinrichtung, Blutbehandlungsvorrichtung zum Aufnehmen einer erfindungsgemäßen externen Funktionseinrichtung, sowie Verfahren"* offenbart.

Die erfindungsgemäße Behandlungsvorrichtung kann wenigstens eine derartige externe Funktionseinrichtung, bevorzugt in Form einer solchen Blutkassette, aufweisen.

Einzelne Komponenten einer Behandlungsvorrichtung gemäß der vorliegenden Erfindung können den in der von der Anmelderin der vorliegenden Erfindung am 11. Februar 2009 beim DPMA hinterlegten Anmeldung 10 2009 008 346.4 mit dem Titel "Verfahren zum *Entfernen von Blut aus einem extrakorporalen Blutkreislauf für eine Behandlungsvorrichtung nach Beenden einer Blutbehandlungssitzung und Vorrichtung zum Ausführen desselben"* offenbarten Ausführungsformen entsprechen.

In einer bevorzugten Ausführungsform weist die Behandlungsvorrichtung wenigstens eine Steuer- oder Regeleinrichtung auf, die dazu geeignet und vorgesehen (also konfiguriert) ist, eine Förderleistung oder eine Förderrate der Blutpumpe und/oder der zweiten Fördereinrichtung unter Berücksichtigung wenigstens eines Parameters den extrakorporalen Blutkreislauf betreffend zu steuern oder zu regeln.

Der Parameter, beispielsweise Daten oder Informationen in Form von Kenngrößen oder Eigenschaften den extrakorporalen Blutkreislauf betreffend, kann ausgewählt sein aus Parametern, die ein Innenvolumen des arteriellen Leitungsabschnitts des extrakorporalen Blut betreffen, Parametern, die ein Innenvolumen des venösen Leitungsabschnitts des extrakorporalen Blutkreislaufs betreffen, Parametern, die eine Zugabestelle für die Substituatflüssigkeit, wie die Lage einer Prädilutions-Zugabestelle für die Substituatflüssigkeit und/oder die Lage einer Postdilutions-Zugabestelle für die Substituatflüssigkeit, betreffen und/oder Parametern, welche die Förderleistung oder Förderrate der zweiten Fördereinrichtung betreffen, ohne jedoch auf die vorstehend genannten Beispiele beschränkt zu sein.

In einer weiteren bevorzugten Ausführungsform kann die Behandlungsvorrichtung wenigstens eine Speichereinrichtung aufweisen, in welcher der Parameter den extrakorporalen Blutkreislauf betreffend speicherbar oder hinterlegbar ist.

Bei der "Speichereinrichtung" kann es sich um eine permanent vorhandene Speichereinrichtung, wie beispielsweise eine Festplatte, und/oder um eine externe Speichereinrichtung, wie eine CD, eine Diskette, eine SD-Karte bzw. SD Memory Card (Secure Digital Memory Card) und dergleichen, handeln.

Die Behandlungsvorrichtung kann wenigstens eine Eingabeeinrichtung aufweisen, mittels welcher der Parameter in einer Speichereinrichtung hinterlegbar ist.

Eine solche "Eingabeeinrichtung" kann eine Tastatur, ein Touch-Pad, ein Touch-Screen, ein Mikrophon, ein Lesegerät und dergleichen sein.

Das Eingeben des Parameters kann durch einen Benutzer, zum Beispiel manuell durch Eintippen in eine Tastatur, erfolgen. Das Hinterlagen des Parameters in der Speichereinrichtung kann auch mittels Übertragung von einem externen Datenträger und/oder einer anderen Speichereinrichtung, beispielsweise über eine IR-Schnittstelle, Bluetooth, einen USB-Port, Firewire, SATA und dergleichen in die Behandlungseinrichtung erfolgen.

Nach Eingeben bzw. Hinterlegen des Parameters in der Speichereinrichtung kann der Parameter dauerhaft abgespeichert werden. Er kann jedoch auch nur für die Dauer einer Behandlungssitzung hinterlegt und beispielsweise temporär gespeichert werden.

In einer weiteren bevorzugten Ausführungsform ist die Speichereinrichtung geeignet und vorgesehen bzw. konfiguriert, Parameter verschiedener und auch unterschiedlicher extrakorporaler Blutkreisläufe zu speichern. Die für das in einer Behandlungssitzung eingesetzte Schlauchsystem bzw. den Blutschlauchsatz gespeicherten Daten können beispielsweise vor Behandlungsbeginn ausgewählt werden.

Dies kann insbesondere vorteilhaft sein, wenn verschiedene oder unterschiedliche Arten oder Systeme oder Anordnungen extrakorporaler Blutkreisläufe bzw. von Schlauchsystemen in bzw. an einer Behandlungsvorrichtung eingesetzt werden sollen.

In einer weiter bevorzugten Ausführungsform kann die Speichereinrichtung an oder in wenigstens einem Abschnitt des extrakorporalen Blutkreislaufs vorgesehen sein. Beispielsweise kann die Speichereinrichtung als Barcode und/oder 2D-Code (Dot-Matrix-Code) und/oder RFID-Chip ausgestaltet sein. Die Behandlungsvorrichtung kann die Daten bzw. Parameter so beispielsweise automatisch einlesen und der Steuer- oder Regeleinrichtung zur Verfügung stellen. Sie kann beispielsweise am Schlauchsystem, z.B. am arteriellen und/oder venösen Leitungsabschnitt des extrakorporalen Blutkreislaufs, vorgesehen sein.

Die Speichereinrichtung kann an oder in einer externen Funktionseinrichtung, wie beispielsweise der vorstehend erwähnten Blutkassette, vorgesehen sein. In der Speichereinrichtung können dann zum Beispiel die während der Herstellung der Blutkassette ermittelten Spezifikationen der einzelnen Komponenten bzw. Abschnitte der Blutkassette hinterlegt sein.

Wenn die Speichereinrichtung als externe Speichereinrichtung vorgesehen ist, kann die Behandlungsvorrichtung vorzugsweise ferner wenigstens eine Aufnahmeeinrichtung und/oder wenigstens eine Leseeinrichtung aufweisen, die zum Aufnehmen und Auslesen der externen Speichereinrichtung geeignet und vorgesehen ist.

Bei der "Aufnahmeeinrichtung" kann es sich zum Beispiel um ein Kartenlesegerät, einen Slot zum Aufnehmen einer SD-Karte oder einer Diskette, einen Einschub zum Aufnehmen einer CD und dergleichen handeln. Die auf der Speichereinrichtung hinterlegten Parameter bzw. Daten bzw. Informationen können nach Einbringen bzw. Einführen bzw. Einschieben der Speichereinrichtung in die Aufnahmeeinrichtung ausgelesen bzw. abgelesen werden. Dies kann mittels einer Leseeinrichtung, wie beispielsweise einem Laser, erfolgen.

Bei der "Leseeinrichtung" kann es sich auch um eine externe Einrichtung bzw. eine exponierte Einrichtung der Behandlungsvorrichtung handeln, wie beispielsweise einen Scanner. Das Einführen bzw. Einschieben oder Einbringen der Speichereinrichtung in eine Aufnahmeeinrichtung ist somit nicht erforderlich. Die Parameter bzw. Daten oder Informationen können durch einfaches Anordnen bzw. Dagegenhalten der Speichereinrichtung vor die Leseeinrichtung aus- bzw. abgelesen werden.

Eine geeignete externe Speichereinrichtung kann beispielsweise auch in oder auf einer Patientenkarte, beispielsweise in Form eines Chips, vorgesehen sein.

Die ausgelesenen bzw. abgelesenen Parameter können an einen zentralen Prozessor einer Steuer- oder Regeleinrichtung der Behandlungsvorrichtung übermittelt und beispielsweise zum Steuern oder Regeln der Förderleistung oder Förderrate der Blutpumpe eingesetzt werden.

In bestimmten erfindungsgemäßen Ausführungsformen weist die Behandlungsvorrichtung einen extrakorporalen Blutkreislauf auf.

Mittels der erfindungsgemäßen Vorrichtung kann Blut, das sich nach Beenden einer Blutbehandlungssitzung in einem extrakorporalen Blutkreislauf der Behandlungsvorrichtung befindet, vorteilhaft zügig aus sowohl dem arteriellen Leitungsabschnitt als auch dem venösen Leitungsabschnitt des extrakorporalen Blutkreislaufs entfernt werden.

Dabei kann in bestimmten erfindungsgemäßen Ausführungsformen eine erhöhte Sicherheit für den Patienten geboten werden. Insbesondere kann eine Gerinnselverschleppung in das Gefäßsystem des Patienten verhindert werden. Zumindest kann die Gefahr einer solchen Verschleppung verringert werden.

Die erfindungsgemäße Behandlungsvorrichtung kann mittels der Steuereinrichtung in der Lage sein, auf Basis der Daten der Innenvolumina aller oder einiger Abschnitte des Blutschlauchsatzes, ggf. unter Berücksichtigung der Lage der gewählten Zugabestelle für das Substituat, und/oder der vorgegebenen Förderrate oder Förderleistung der Substituatpumpe, die erforderliche Förderrate oder die erforderliche Förderleistung der Blutpumpe oder einer anderen Fördereinrichtung automatisch derart zu berechnen und die Blutpumpe oder andere Fördereinrichtung entsprechend zu steuern, dass eine gleichzeitige Verdrängung des Blut aus den i.d.R. unterschiedlichen Volumina des arteriellen und des venösen Leitungsabschnitts in den Patienten erfolgen kann.

Besonders vorteilhaft kann die Blutpumpe oder eine andere Fördereinrichtung derart gesteuert werden, dass die Blutrückgabe nicht nur gleichzeitig, sondern auch gleichschnell erfolgen kann. Erfindungsgemäß kann es dadurch vorteilhaft möglich sein, das Entfernen des Bluts aus beiden Leitungsabschnitten des extrakorporalen Blutkreislaufs gleichzeitig abzuschließen. Auf diese Weise können auf besonders vorteilhafte Weise Zeit und Kosten eingespart werden.

Da das Blut gleichzeitig und mit gleichem Fortschritt aus den beiden Leitungsabschnitten in das Gefäßsystem des Patienten rückgeführt werden kann, kann vorteilhaft eine unnötige oder ungewollte Infusion von Substituatflüssigkeit nach Beenden der Blutbehandlung in das Gefäßsystem des Patienten vermieden werden.

Da das Blut im Wesentlichen vollständig aus dem extrakorporalen Blutkreislauf entfernt und der extrakorporale Blutkreislauf gleichzeitig mit Substituatflüssigkeit gespült werden kann, kann vorteilhaft die Gefahr einer Kontamination Dritter oder der Umgebung mit Patientenblut vermindert werden. Dies gilt insbesondere für die Entsorgung des extrakorporalen Blutkreislaufs nach Beendigung der Behandlung.

Im Folgenden wird die vorliegende Erfindung anhand bevorzugter Ausführungsformen derselben unter Bezugnahme auf die Zeichnung beschrieben. In der Zeichnung bezeichnen identische Bezugszeichen gleiche oder identische Elemente. Es gilt:
- Fig. 1: zeigt eine schematische Übersicht einer beispielhaften erfindungsgemäßen Behandlungsvorrichtung zur Durchführung des Verfahrens, das nicht beansprucht wird;
- Fig. 2: zeigt schematisch vereinfacht eine Phase während der Durchführung eines erfindungsgemäßen Verfahrens, das nicht beansprucht wird; und
- Fig. 3: zeigt eine Prinzipskizze eines extrakorporalen Blutsystems mit arteriellem ABD (Air Bubble Detector).

Zur beispielhaften Erläuterung der vorliegenden Erfindung ist als Behandlungsvorrichtung eine Blutbehandlungsvorrichtung, beispielsweise eine Hämodialysiervorrichtung, ausgewählt. Es wird ausdrücklich darauf hingewiesen, dass die Entleerung des extrakorporalen Blutkreislaufs auch gemäß der Erfindung erfolgen kann ohne dass das entleerte Blut dem Patienten rückgeführt wird.

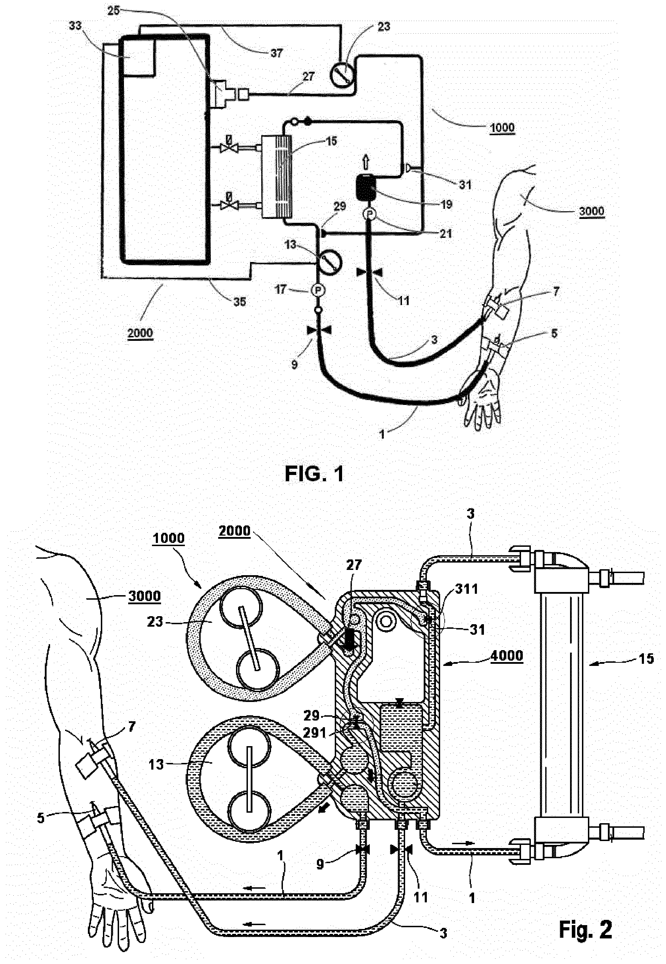

Die normalen Pfeile in der Figur 2 zeigen eine Richtung des Blutstroms an. Die Blockpfeile zeigen jeweils eine Richtung des Substituatstroms an.

In **Fig.** 1 ist ein extrakorporaler Blutkreislauf 1000 einer erfindungsgemäßen Behandlungsvorrichtung 2000 mit einem DoubleNeedle-Zugang zum Gefäßsystem eines Patienten 3000 dargestellt. Der extrakorporale Blutkreislauf 1000 weist einen arteriellen Leitungsabschnitt 1 sowie einen venösen Leitungsabschnitt 3 auf.

Der arterielle Leitungsabschnitt 1 ist über einen arteriellen Patientenzugang, beispielsweise, wie in Fig. 1 gezeigt, in Form einer arteriellen Konnektionsnadel 5, mit dem Gefäßsystem des Patienten 3000 verbunden. Der venöse Leitungsabschnitt 3 ist über einen venösen Patientenzugang, beispielsweise, wie in Fig. 1 gezeigt, in Form einer venösen Konnektionsnadel 7, mit dem Gefäßsystem des Patienten 3000 verbunden.

Der arterielle Leitungsabschnitt 1 weist eine arterielle Patientenschlauchklemme 9 auf, der venöse Leitungsabschnitt 3 weist eine venöse Patientenschlauchklemme 11 auf. In Fig. 1 sind sowohl die arterielle Patientenschlauchklemme 9 als auch die venöse Patientenschlauchklemme 11 in einem geöffneten bzw. offenen Zustand dargestellt.

Der extrakorporale Blutkreislauf 1000 weist eine Blutpumpe 13 auf. Die Blutpumpe 13 ist exemplarisch als Rollenpumpe ausgestaltet. Die Blutpumpe 13 fördert Blut des Patienten 3000 durch den extrakorporalen Blutkreislauf 1000.

Der extrakorporale Blutkreislauf 1000 weist eine Blutbehandlungseinrichtung 15, beispielsweise einen Hämodialysator, auf.

Wie in Fig. 1 gezeigt, ist im arteriellen Leitungsabschnitt 1 ein arterieller Drucksensor 17 stromabwärts (bezogen auf die während einer Blutbehandlung übliche Förderrichtung) von der Blutpumpe 13 angeordnet.

Im venösen Leitungsabschnitt 3 sind eine venöse Tropfkammer 19 und ein venöser Drucksensor 21 stromaufwärts der venösen Tropfkammer 19 angeordnet.

Mittels einer zweiten Fördereinrichtung, beispielsweise einer Substituatpumpe 23, kann Substituatflüssigkeit aus einer Substituatflüssigkeitsvorratseinrichtung 25 über eine Substituatsflüssigkeitszuleitung 27 an einer Prädilutions-Zugabestelle 29 für Substituatflüssigkeit und/oder einer Postdilutions-Zugabestelle 31 für Substituatflüssigkeit in den extrakorporalen Blutkreislauf 1000 eingebracht werden. Die Substituatpumpe 23 ist hier exemplarisch als Rollenpumpe ausgestaltet.

Die Behandlungsvorrichtung 2000 weist eine Regel- oder Steuereinrichtung 33 auf. Die Steuereinrichtung 33 ist über eine Steuerleitung 35 mit der Blutpumpe 13 und über eine Steuerleitung 37 mit der Substituatpumpe 23 verbunden. Es kann allerdings genügen, nur eine Regel- oder Steuerleitung, wahlweise zur Blutpumpe 13 oder zur Substituatpumpe 23 vorzusehen zum Ausführen des Verfahrens, das nicht beansprucht wird.

Die Steuereinrichtung 33 kann beispielsweise eine Förderrate oder eine Förderleistung der Blutpumpe 13 in Abhängigkeit von Betriebsparametern der Substituatpumpe 23, z.B. deren Förderrate oder Förderleistung, steuern.

Die Steuerleitungen 35 und 37 können eine bidirektionale Kommunikation zwischen der Steuereinrichtung 33 und der Blutpumpe 13 bzw. der Substituatpumpe 23 zulassen.

Fig. 1 zeigt eine Verbindung der erfindungsgemäßen Blutbehandlungsvorrichtung 2000 mit dem Gefäßsystem des Patienten. Eine solche Verbindung ist zur Verwirklichung der erfindungsgemäßen Blutbehandlungsvorrichtung jedoch nicht zwingend erforderlich, sondern stellt nur eine besondere Ausgestaltung und eine bevorzugte Nutzung dar.

In Fig. 2 weist die Behandlungsvorrichtung 2000 eine externe Funktionseinrichtung, wie beispielsweise eine Blutkassette 4000, auf. Die Blutkassette 4000 kann eine Blutkassette sein, wie sie in den von der Anmelderin der vorliegenden Erfindung am 23.April 2009 und am 10. Juni 2009 beim DPMA hinterlegten Anmeldungen mit jeweils dem Titel "*Externe Funktionseinrichtung, Blutbehandlungsvorrichtung zum Aufnehmen einer erfindungsgemäßen externen Funktionseinrichtung, sowie Verfahren*", siehe oben, offenbart ist.

Fig. 2 zeigt eine Phase eines Verfahrens, das nicht beansprucht wird, zum Entfernen von Blut aus dem extrakorporalen Blutkreislauf 1000 nach Beendigung einer Blutbehandlung in einer besonderen Ausführungsform, bei welcher das Blut in das Gefäßsystem des Patienten rückgeführt wird.

Sowohl die arterielle Patientenschlauchklemme 9 als auch die venöse Patientenschlauchklemme 11 befinden sich in einem geöffneten bzw. offenen Zustand.

Die Blutpumpe 13 und die Substituatpumpe 23 werden betrieben.

Im Folgenden beziehen sich die Begriffe "Uhrzeigersinn" oder "gegen den Uhrzeigersinn" auf die Zeichenebene der Fig. 2. Sie sollen dazu dienen, die Förderrichtung der Pumpen während einer Dialysebehandlung und während des Verfahrens, das nicht beansprucht wird, anzugeben: Während eines Blutbehandlungsverfahrens fördern die Blutpumpe 13 und die Substituatpumpe 23 gewöhnlich gegen den Uhrzeigersinn, wie er Fig. 2 zu entnehmen wäre.

Während der Durchführung des Verfahrens, das nicht beansprucht wird, rotiert die Blutpumpe 13 im Uhrzeigersinn und damit gegen die während einer Blutbehandlung übliche Förderrichtung (Patient - arterieller Leitungsabschnitt - Blutbehandlungseinrichtung - venöser Leitungsabschnitt - Patient).

Die Substituatpumpe 23 rotiert gegen den Uhrzeigersinn.

Die Rotationsgeschwindigkeiten der Blutpumpe 13 und der Substituatpumpe 23 können voneinander verschieden sein. Die Rotationsgeschwindigkeiten der Blutpumpe 13 und der Substituatpumpe 23 können identisch sein. Die Blutpumpe 13 und die Substituatpumpe 23 können zum gleichen Zeitpunkt gestartet werden und/oder für eine gleiche (vorgegebene) Zeitdauer rotieren gelassen werden. Die Blutpumpe 13 und die Substituatpumpe 23 können zu verschiedenen Zeitpunkten und/oder verschiedene (vorgegebene) Zeitdauern lang rotieren gelassen werden.

Im extrakorporalen Blutkreislauf 1000 befindet sich Blut (einfach schraffierter Bereich). Über die Substituatflüssigkeitszuleitung 27 wird Substituatflüssigkeit eingebracht (mit Kreuzmuster schraffierter Bereich in Fig. 2).

Ein Prädilutions-Zugabeventil 291 der Prädilutions-Zugabestelle 29 ist geöffnet. Alternativ oder zusätzlich kann auch ein Postdilutions-Zugabeventil 311 der Postdilutions-Zugabestelle 31 geöffnet sein. In der in Fig. 2 gezeigten Darstellung ist das Postdilutions-Zugabeventil 311 der Postdilutions-Zugabestelle 31 jedoch geschlossen.

Das Prädilutions-Zugabeventil 291 und das Postdilutions-Zugabeventil 311 können beispielsweise als so genannte Phantomventile ausgestaltet sein, wie sie in den o.g. Anmeldungen der vorliegenden Anmelderin mit dem Titel *"Externe Funktionseinrichtung, Blutbehandlungsvorrichtung zum Aufnehmen einer erfindungsgemäßen externen Funktionseinrichtung, sowie Verfahren"* sowie in der Anmeldung 10 2009 012 632.5 (mit dem Titel *"Abdichtungseinrichtung zum Abdichten eines Volumens einer medizinischen Behandlungsanordnung gegen ein weiteres Volumen sowie Anordnung und Verfahren",* eingereicht am 10. März 2009 beim DPMA, offenbart sind.

Über das Prädilutions-Zugabeventil 291 kann Substituatflüssigkeit in den extrakorporalen Blutkreislauf 1000 eintreten.

Fig. 2 zeigt die Konfiguration der Blutbehandlungsvorrichtung 2000 in einer Momentaufnahme während des Blutentleerungs- bzw. Blutrückführungsprozesses, bei welcher die Substituatflüssigkeit durch das geöffnete Prädilutions-Zugabeventil 291 stromaufwärts (bezogen auf die während einer Blutbehandlung übliche Förderrichtung) in den extrakorporalen Blutkreislauf 1000 eingebracht wird.

Die Substituatflüssigkeit kann das Blut zwischen dem Prädilutions-Zugabeventil 291 und der venösen Konnektionsnadel 7, also das im stromaufwärts des Prädilutions-Zugabeventils 291 befindliche Blut im arteriellen Leitungsabschnitt 1 und das im venösen Leitungsabschnitt 3 befindliche Blut, verdrängen.

Das verdrängte Blut kann durch die venöse Konnektionsnadel 7 in das Gefäßsystem des Patienten rückgeführt werden.

Die Substituatflüssigkeit wird durch den arteriellen Leitungsabschnitt 1 des extrakorporalen Blutkreislaufs 1000 stromaufwärts des Prädilutions-Zugabeventils 291 durch die Behandlungseinrichtung 15 und den venösen Leitungsabschnitt 3 zum Patienten 3000 hin gefördert.

Gleichzeitig wird das im extrakorporalen Blutkreislauf 1000 in Förderrichtung vor dem bzw. stromabwärts des Prädilutions-Zugabeventils 291 befindliche Blut durch den arteriellen Leitungsabschnitt 1 zum Patienten 3000 gefördert.

Die Substituatflüssigkeit wird dabei vom Prädilutions-Zugabeventil 291 aus in den stromabwärts vom Prädilutions-Zugabeventil 291 angeordneten arteriellen Leitungsabschnitt 1 eingebracht und in Richtung des Patienten 3000 gefördert.

Das gleichzeitige Entleeren des Bluts aus dem arteriellen Leitungsabschnitt 1 des extrakorporalen Blutkreislaufs 1000 und dem venösen Leitungsabschnitt 3 des extrakorporalen Blutkreislaufs 1000 kann, wie es bevorzugt ist, mit gleichem Fortschritt erfolgen, d.h. das Ausmaß der Entleerung (z.B. in Prozent eines Leitungsinhalts) des sich jeweils noch in den Leitungsabschnitten 1 und 3 befindlichen Bluts kann zu jedem Zeitpunkt identisch sein.

Das Entleeren bzw. Rückführen des Bluts aus dem extrakorporalen Blutkreislauf 1000 kann gleichzeitig beendet werden. Zum Beispiel können, kurz bevor die Substituatflüssigkeit die arterielle Konnektionsnadel 5 erreicht und entsprechend kurz bevor die Substituatflüssigkeit die venöse Konnektionsnadel 7 erreicht, die Blutpumpe 13 und die Substituatpumpe 23 gestoppt werden.

Alternativ kann die Substituatflüssigkeit, wie vorstehend bereits erwähnt, auch oder zusätzlich auch über das Postdilutions-Zugabeventil 311 in den extrakorporalen Blutkreislauf 1000 eingebracht werden.

Die Durchführung des Verfahrens, das nicht beansprucht wird, entspricht aber im Wesentlichen der vorstehend für mittels Prädilution zugeführte Substituatflüssigkeit angegebenen Vorgehensweise, so dass an dieser Stelle auf die obigen Ausführungen verwiesen wird.

Fig. 3 zeigt eine Prinzipskizze eines extrakorporalen Blutsystems mit arteriellem ABD (Air Bubble Detector) bzw. Lufterkennungsdetektor zum Ausführen von Verfahren, die nicht beansprucht werden. Die dargestellten Komponenten sind im Wesentlichen bereits aus den Fig. 1 und 2 bekannt. Gegenüber diesen hinzugekommen sind lediglich eine Hydraulik 39, ein arterieller ABD (Air Bubble Detector)41 und ein venöser ABD 43.

Die mit durchgezogenen Pfeilen angegebenen Pumprichtungen entsprechend den regulären Richtungen oder den Vorwärtsrichtungen. Die mit Punktlinien angegebene Pumprichtung der Blutpumpe 13 entspricht deren Rückwärtslauf oder einem Ansaugen durch die Blutpumpe 13.

## Patentansprüche

1. Behandlungsvorrichtung (2000) mit
- wenigstens einer Einrichtung zum Aufnehmen wenigstens eines extrakorporalen Blutkreislaufs (1000), umfassend einen arteriellen Leitungsabschnitt (1) mit einem Leitungsinneren und einen venösen Leitungsabschnitt (3) mit einem Leitungsinneren;
- wenigstens einer an oder in dem extrakorporalen Blutkreislauf (1000) angeordneten Blutpumpe (13) zum Fördern von Blut innerhalb des extrakorporalen Blutkreislaufs (1000);
- wenigstens einer zweiten Fördereinrichtung zum Einbringen wenigstens einer Flüssigkeit in den extrakorporalen Blutkreislauf (1000) und/oder zum Fördern der Flüssigkeit im extrakorporalen Blutkreislauf (1000);
**gekennzeichnet durch**
- wenigstens eine Steuer- oder Regeleinrichtung (33), die geeignet und vorgesehen ist zum Ausführen eines Verfahrens zum Entfernen von Blut nach Beenden einer Blutbehandlungssitzung mit den Schritten:
- Einbringen von Flüssigkeit in den extrakorporalen Blutkreislauf (1000);
- Fördern der Flüssigkeit zum gleichzeitigen Austragen von Blut sowohl aus dem arteriellen Leitungsabschnitt (1) und als auch aus dem venösen Leitungsabschnitt (3) des extrakorporalen Blutkreislaufs (1000).

2. Behandlungsvorrichtung (2000) nach Anspruch 1, wobei Blut unter gleichzeitigem Betreiben der Blutpumpe (13) und der zweiten Fördereinrichtung aus dem extrakorporalen Blutkreislauf (1000) gefördert wird.

3. Behandlungsvorrichtung (2000) nach Anspruch 1 oder 2, wobei Blut mit gleich schnellem relativem Fortschritt sowohl aus dem arteriellen Leitungsabschnitt (1) als auch dem venösen Leitungsabschnitt (3) des extrakorporalen Blutkreislaufs (1000) gefördert wird.

4. Behandlungsvorrichtung (2000) nach Anspruch 1 oder 2, wobei das Blut mit unterschiedlichem relativem Fortschritt sowohl aus dem arteriellen Leitungsabschnitt (1) als auch dem venösen Leitungsabschnitt (3) des extrakorporalen Blutkreislaufs (1000) gefördert wird.

5. Behandlungsvorrichtung (2000) nach einem der vorangegangenen Ansprüche, welche wenigstens eine in einem Abschnitt des extrakorporalen Blutkreislaufs (1000) angeordnete Erfassungseinrichtung zum Erfassen einer qualitativen Änderung des Inhalts des extrakorporalen Blutkreislaufs aufweist, wobei das Verfahren die Schritte:
- Fördern von Blut, bis eine Phasengrenze oder ein Mischbereich zwischen Blut und Flüssigkeit von der Erfassungseinrichtung erkannt wird; und
- Beenden des Förderns des Bluts bei Erkennen der Phasengrenze oder des Mischbereichs durch die Erfassungseinrichtung,
aufweist.

6. Behandlungsvorrichtung (2000) nach einem der vorangegangenen Ansprüche mit wenigstens einer Steuer- oder Regeleinrichtung (33), die dazu geeignet und vorgesehen ist, eine Förderleistung oder eine Förderrate der Blutpumpe (13) und/oder der zweiten Fördereinrichtung unter Berücksichtigung wenigstens eines Parameters den extrakorporalen Blutkreislauf (1000) betreffend zu steuern oder zu regeln.

7. Behandlungsvorrichtung (2000) nach Anspruch 6, wobei der Parameter den extrakorporalen Blutkreislauf (1000) betreffend ausgewählt ist aus Parametern, die ein Innenvolumen des arteriellen Leitungsabschnitts (1) des extrakorporalen Blutkreislaufs (1000) betreffen, Parametern, die ein Innenvolumen des venösen Leitungsabschnitts (3) des extrakorporalen Blutkreislaufs (1000) betreffen, Parametern, die eine Zugabestelle für eine Substituatflüssigkeit, wie die Lage einer Prädilutions-Zugabestelle (29) für die Substituatflüssigkeit und/oder die Lage einer Postdilutions-Zugabestelle (31) für die Substituatflüssigkeit, betreffen und/oder Parametern, welche die Förderleistung oder die Förderrate der zweiten Fördereinrichtung betreffen.

8. Behandlungsvorrichtung (2000) nach einem der Ansprüche 6 bis 7, wobei die Behandlungsvorrichtung (2000) ferner wenigstens eine Speichereinrichtung aufweist, welche ausgestaltet und vorgesehen ist, den Parameter zu speichern.

9. Behandlungsvorrichtung (2000) nach einem der Ansprüche 6 bis 8, wobei die Behandlungsvorrichtung (2000) ferner wenigstens eine Eingabeeinrichtung aufweist, mittels welcher der Parameter in einer Speichereinrichtung hinterlegbar ist.

10. Behandlungsvorrichtung (2000) nach einem der Ansprüche 1 bis 9, wobei die Behandlungsvorrichtung (2000) ferner wenigstens eine externe Funktionseinrichtung, insbesondere eine Blutkassette (4000), aufweist.

11. Behandlungsvorrichtung (2000) nach einem der Ansprüche 1 bis 10, wobei die Behandlungsvorrichtung eine Dialysiervorrichtung oder eine Hämodialysiervorrichtung ist.

12. Behandlungsvorrichtung (2000) nach einem der Ansprüche 1 bis 11, wobei die Blutpumpe (13) als Rollenpumpe ausgestaltet ist.

13. Behandlungsvorrichtung (2000) nach einem der Ansprüche 1 bis 12, wobei die zweite Fördereinrichtung als Rollenpumpe ausgestaltet ist.

14. Behandlungsvorrichtung (2000) nach einem der Ansprüche 1 bis 13 mit wenigstens einem extrakorporalen Blutkreislauf (1000).

## Claims

1. Treatment apparatus (2000) comprising:
- at least one device for receiving at least one extracorporeal blood circuit (1000), including an arterial conduit portion (1) having a conduit interior and a venous conduit portion (3) having a conduit interior;
- at least one blood pump (13) arranged on or in the extracorporeal blood circuit (1000) for conveying blood within the extracorporeal blood circuit (1000);
- at least one second conveying means for introducing at least one liquid into the extracorporeal blood circuit (1000) and/or for conveying the liquid in the extracorporeal blood circuit (1000);
**characterized by**:
- at least one control or regulating device (33) suitable and provided for carrying out a method for removing blood after finishing a blood treatment session comprising the steps of:
- introducing liquid into the extracorporeal blood circuit (1000);
- conveying the fluid for simultaneously discharging blood from both the arterial conduit portion (1) and the venous conduit portion (3) of the extracorporeal blood circuit (1000).

2. The treatment apparatus (2000) according to claim 1, wherein blood is conveyed from the extracorporeal blood circuit (1000) while simultaneously operating of the blood pump (13) and the second conveying means.

3. The treatment apparatus (2000) according to claim 1 or 2, wherein blood is conveyed with equal velocity relative progress from both the arterial conduit portion (1) and the venous conduit portion (3) of the extracorporeal blood circuit (1000).

4. The treatment apparatus (2000) according to claim 1 or 2, wherein blood is conveyed at different relative progress from both the arterial conduit portion (1) and the venous conduit portion (3) of the extracorporeal blood circuit (1000).

5. The treatment apparatus (2000) according to anyone of the preceding claims, which comprises at least one detection means arranged in a portion of the extracorporeal blood circuit (1000) for detecting a qualitative change in the content of the extracorporeal blood circuit, the method comprising the steps of:
- conveying blood, until a phase boundary or a mixing region between blood and liquid is detected by the detection device; and
- terminating conveyance of blood upon detection of the phase boundary or of the mixing region by the detection means.

6. The treatment apparatus (2000) according to anyone of the preceding claims comprising at least one control or regulating device (33) suited and provided for controlling or regulating a conveying capacity or conveying rate of the blood pump (13) and/or of the second conveying means, under consideration of at least one parameter relating to the extracorporeal blood circuit (1000).

7. The treatment apparatus (2000) according to claim 6, wherein the parameter relating to the extracorporeal blood circuit (1000) is selected from parameters relating to an inner volume of the arterial conduit portion (1) of the extracorporeal blood circuit (1000), parameters relating to an inner volume of the venous conduit portion (3) of the extracorporeal blood circuit (1000), parameters relating to an addition point for a substituate liquid, such as the position of a pre-dilution addition point (29) for the substituate liquid and/or the position of a post-dilution addition point (31) for the substituate liquid,
and/or parameters relating to the conveying capacity or the conveying rate of the second conveying means.

8. The treatment apparatus (2000) according to anyone of claims 6 to 7, wherein the treatment apparatus (2000) further comprises at least one memory means designed and intended to store the parameter.

9. The treatment apparatus (2000) according to anyone of claims 6 to 8, wherein the treatment apparatus (2000) further comprises at least one input means whereby the parameter may be deposited in a memory means.

10. The treatment apparatus (2000) according to anyone of claims 1 to 9, wherein the treatment apparatus (2000) further comprises at least one external functional device, in particular a blood cassette (4000).

11. The treatment apparatus (2000) according to anyone of claims 1 to 10, wherein the treatment apparatus (2000) is a dialysis apparatus or a hemodialysis apparatus.

12. The treatment apparatus (2000) according to anyone of claims 1 to 11, wherein the blood pump (13) is designed as a roller pump.

13. The treatment apparatus (2000) according to anyone of claims 1 to 12, wherein the second conveying means is designed as a roller pump.

14. The treatment apparatus (2000) according to anyone of claims 1 to 13 comprising at least one extracorporeal blood circuit (1000).

## Revendications

1. Un appareil de traitement (2000) comprenant:
- au moins un dispositif destiné à recevoir au moins un circuit sanguin extracorporel (1000), comportant une section de conduit artériel (1) avec un intérieur de conduit ainsi qu'une section de conduit veineux (3) avec un intérieur de conduit;
- au moins une pompe à sang (13) agencée sur ou dans le circuit sanguin extracorporel (1000) pour l'acheminement du sang à l'intérieur du circuit sanguin extracorporel (1000);
- au moins un second moyen d'acheminement pour introduire au moins un liquide dans le circuit sanguin extracorporel (1000) et/ou pour acheminer le liquide dans le circuit sanguin extracorporel (1000);
**caractérisé par**:
- au moins un dispositif de commande ou de régulation (33) adapté et prévu pour exécuter un procédé d'élimination du sang après la fin d'une séance de traitement du sang, comprenant les étapes consistant à:
- l'introduction de liquide dans le circuit sanguin extracorporel (1000);
- l'acheminement de liquide pour l'évacuation simultanée du sang à la fois de la section de conduit artériel (1) et de la section de conduit veineux (3) du circuit sanguin extracorporel (1000).

2. L'appareil de traitement (2000) selon la première revendication, où le sang est acheminé hors du circuit sanguin extracorporel (1000) tout en actionnant simultanément la pompe à sang (13) et le second moyen d'acheminement.

3. L'appareil de traitement (2000) selon la revendication 1 ou 2, où le sang est acheminé avec la même vitesse de progression relative aussi bien depuis la section de conduit artériel (1) que depuis la section de conduit veineux (3) du circuit sanguin extracorporel (1000).

4. L'appareil de traitement (2000) selon la revendication 1 ou 2, où le sang est acheminé avec une progression relative différente aussi bien depuis la section de conduit artériel (1) que depuis la section de conduit veineux(3) du circuit sanguin extracorporel (1000).

5. L'appareil de traitement (2000) selon l'une quelconque des revendications précédentes, qui comprend au moins un dispositif de détection agencé dans une partie du circuit sanguin extracorporel (1000) pour détecter un changement qualitatif du contenu du circuit sanguin extracorporel, le procédé comprenant les étapes consistant à:
- l'acheminement du sang jusqu'à ce qu'une limite de phase ou une zone de mélange entre le sang et le liquide soit détectée par le dispositif de détection; et
- l'arrêt de l'acheminement du sang dès la détection de la limite de phase ou de la zone de mélange par le dispositif de détection.

6. L'appareil de traitement (2000) selon l'une quelconque des revendications précédentes comprenant au moins un dispositif de commande ou de régulation (33) adapté et prévu pour commander ou réguler un débit d'acheminement ou un taux d'acheminement de la pompe à sang (13) et/ou du second dispositif d'acheminement, en tenant compte d'au moins un paramètre relatif au circuit sanguin extracorporel (1000).

7. L'appareil de traitement (2000) selon la revendication 6, où le paramètre relatif au circuit sanguin extracorporel (1000) est sélectionné parmi des paramètres relatifs à un volume interne de la section de conduit artériel (1) du circuit sanguin extracorporel (1000), des paramètres relatifs à un volume interne de la section de conduit veineux (3) du circuit sanguin extracorporel (1000), des paramètres relatifs à un point d'addition pour un liquide de substitut, tels que l' emplacement d'un point d'addition de prédilution (29) pour le liquide de substitut et/ou l'emplacement d'un point de postdilution (31) pour le liquide de substitut, et/ou des paramètres relatifs au débit d'acheminement ou aux taux d'acheminement du second dispositif d'acheminement.

8. L'appareil de traitement (2000) selon l'une quelconque des revendications 6 à 7, où l'appareil de traitement (2000) comprend en outre au moins un dispositif de mémorisation conçu et prévu pour enregistrer le paramètre.

9. L'appareil de traitement (2000) selon l'une quelconque des revendications 6 à 8, où l'appareil de traitement (2000) comprend en outre au moins un dispositif d'entrée au moyen duquel le paramètre peut être déposé dans un dispositif de mémorisation.

10. L'appareil de traitement (2000) selon l'une quelconque des revendications 1 à 9, où l'appareil de traitement (2000) comprend en outre au moins un dispositif fonctionnel externe, notamment une cassette à sang (4000).

11. L'appareil de traitement (2000) selon l'une quelconque des revendications 1 à 10, où l'appareil de traitement (2000) est un appareil de dialyse ou un appareil d'hémodialyse.

12. L'appareil de traitement (2000) selon l'une quelconque des revendications 1 à 11, où la pompe à sang (13) est conçue comme une pompe à galets.

13. L'appareil de traitement (2000) selon l'une quelconque des revendications 1 à 12, où le second dispositif d'acheminement est conçu comme une pompe à galets.

14. L'appareil de traitement (2000) selon l'une quelconque des revendications 1 à 13 comprenant au moins un circuit sanguin extracorporel (1000).
